# EUROPÄISCHE PATENTSCHRIFT

(11) **EP 0 695 163 B1**
(45) Veröffentlichungstag und Bekanntmachung des Hinweises auf die Patenterteilung: **05.03.1997**
(21) Anmeldenummer: 94915085.8
(22) Anmeldetag: 21.04.1994
(51) Int. Cl.: A61K 7/13

(54) **ISATINDERIVATE ZUM FÄRBEN VON KERATINHALTIGEN FASERN**
ISATIN DERIVATIVES FOR DYEING KERATIN-CONTAINING FIBRES
DERIVES D'ISATINE PERMETTANT DE COLORER DES FIBRES CONTENANT DE LA KERATINE

(30) Priorität: 30.04.1993 DE 4314318
(43) Veröffentlichungstag der Anmeldung: 07.02.1996
(73) Patentinhaber: Henkel Kommanditgesellschaft auf Aktien, 40191 Düsseldorf (DE)
(72) Erfinder: MÖLLER, Hinrich, D-40789 Monheim (DE); HÖFFKES, Horst, D-40595 Düsseldorf (DE)
(86) Internationale Anmeldenummer: EP9401247
(87) Internationale Veröffentlichungsnummer: WO9424989

(56) Entgegenhaltungen:
- WO-A-93/19725
- S.T.N, File Supplier, Karlsruhe, DE, File Chemical Abstracts, vol 93:130671 siehe die zusammenfassung
- S.T.N., File Supplier, Karlsruhe, DE, File Chemical Abstracts, vol 114, n 108694 siehe die zusammenfassung & JP-A-02231414, KASHIWA CHEMICL CO., 9-13-90

## Beschreibung

Gegenstand der Erfindung ist die Verwendung von bestimmten sulfo- oder carboxylgruppenhaltigen Isatinderivaten zum Färben von keratinhaltigen Fasern sowie diese Isatinderivate enthaltende Färbemittel.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von Oxidationsmitteln wie z. B. H₂O₂, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Farbesysteme auf Basis von Isatin oder Isatinderivaten bieten hier eine Alternative. Isatin ist als Direktfarbstoff zum Färben von Keratinfasern alleine oder in Kombination mit Chinonfarbstoffen in der deutschen Offenlegungsschrift DE 36 35 147 A1 beschrieben worden.
Die Variationsbreite der erzielbaren Nuancen ist jedoch beschränkt. In den allermeisten Fällen erhält man eine goldfarbene Färbung.

Ein weiteres isatinhaltiges Färbesystem wird in der europäischen Offenlegungsschrift EP 359 465 A2 beschrieben. Hier wird die Färbung mit Hilfe eines aus der Reaktion eines Isatins mit einem Anilinderivat entstehenden Ketimins (Schiff'sche Base) erzielt. Das Ketimin wird entweder als solches auf keratinische Fasern aufgebracht und entwickelt dort eine Färbung, oder aber eine aus einem Isatin und einem Anilinderivat bestehende Mischung wird auf die Faser aufgebracht und bildet zunächst "in situ" das Ketimin, woraufhin sich auf der Faser die Färbung entwickelt.

Die europäische Offenlegungsschrift EP 497 697 A1 beschreibt Haarfärbemittel auf Basis von Isatinen und Aminoindolen oder -indolinen mit primärer Aminogruppe, wobei sich in einer Kondensationsreaktion Schiff'sche Basen bilden.

Die europäische Offenlegungsschrift EP 0 502 783 A1 beschreibt Haarfärbemittel, die Isatine und Aminopyridine oder Isatine und Aminopyrimidine mit primärer Aminogruppe enthalten.

Die europäische Offenlegungsschrift EP 0 502 784 A1 beschreibt Haarfärbemittel, die Isatine und substituierte Diamine oder Aminophenole oder aber Isatine und (Bisaryl)-alkylendiamine enthalten.

Überraschenderweise wurde nun gefunden, daß sich bestimmte sulfo- oder carboxylgruppenhaltige Isatinderivate hervorragend zum Färben von keratinhaltigen Fasern eignen.

Als keratinhaltige Fasern kommen z.B. Wolle, Pelze, Felle und menschliche Haare in Betracht. Die unten näher bezeichneten sulfo- oder carboxylgruppenhaltigen Isatinderivate können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl oder Acetylcellulose und synthetischer Fasern, wie z.B. Polyamid-, Polyacrylnitril,, Polyurethang und Polyesterfasern verwendet werden.

Gegenstand der Erfindung ist die Verwendung von Isatinderivaten der Formel I 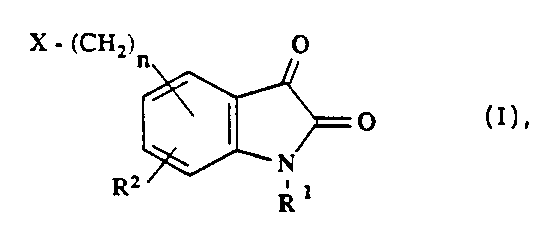 wobei R¹ Wasserstoff, eine C₁-C₄-Alkyl-, C₂-C₄-Hydroxyalkyl-, C₂-C₂₀-Acyl-, Benzyl- oder Phenylgruppe, R² Wasserstoff, eine C₁-C₄-Alkyl-, C₁-C₄-Alkoxy-, Hydroxy-, Halogen- oder Nitrogruppe, X eine Sulfogruppe - SO₃H oder eine Carboxylgruppe - COOH darstellt und n eine ganze Zahl von 0 bis 3 ist, und deren wasserlöslichen Salzen zum Färben von keratinhaltigen Fasern.

Als wasserlösliche Salze sind ohne Einschränkung z.B. die Alkali- oder Ammoniumsalze zu nennen.

Vorzugsweise werden zum Färben von keratinhaltigen Fasern diejenigen Isatinderivate der Formel I verwendet, in denen R¹ und R² Wasserstoffe sind und in denen n gleich 0 ist, d.h. solche Isatinderivate der Formel I, in denen die Sulfo- oder Carboxylgruppe direkt an den aromatischen Ring gebunden ist. Ganz besonders eignen sich Isatin-5-sulfonsäure, Isatin-4-carbonsäure und Isatin-5-carbonsäure.

Die Isatinderivate der Formel I ergaben Nuancen im Gelb-Bereich. Die Waschbeständigkeit der Färbungen ist trotz der Anwesenheit der hydrophilen Sulfo- bzw. Carboxylgruppe überraschend hoch. Gleichzeitig erzielt man in rein wäßrigen Systemen größere Farbtiefen; auf die Verwendung von toxikologisch bedenklichen löslichkeitsvermittelnden Agenzien wie z.B. primäre kurzkettige Alkohole kann dabei verzichtet werden, ohne daß deren Verwendung grundsätzlich auszuschließen ist.

Besonders brillante Färbungen im Gelb-, Rot- und Violettbereich mit guten Echtheitseigenschaften (Lichtechtheit, Waschechtheit, Reibechtheit) werden erzielt, wenn die Isatinderivate der Formel I gemeinsam mit bestimmten aminogruppenhaltigen Verbindungen oder gemeinsam mit bestimmten Indol- oder Indolinderivaten verwendet werden.

Ein weiterer Erfindungsgegenstand sind deshalb Mittel zum Färben von keratinhaltigen Fasern, enthaltend mindestens ein Isatinderivat der Formel I und mindestens eine Aminosäure oder ein aus 2 bis 9 Aminosäuren aufgebautes Oligopeptid.

Als Aminosäuren kommen alle natürlich vorkommenden und synthetischen Aminsäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden.

Geeignete Oligopeptide sind alle aus natürlich vorkommenden und synthetischen Aminosäuren aufgebauten Oligopeptide. Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen.

Zum Einsatz in den erfindungsgemäßen Färbemitteln eignen sich jedoch besonders diejenigen Aminosäuren oder Oligopeptide, die ausgewänlt sind aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

Ein weiterer Erfindungsgegenstand sind Mittel zum Färben von keratinhaltigen Fasern enthaltend mindestens ein Isatinverdivat der Formel I und mindestens ein aromatisches Amin der Formel II 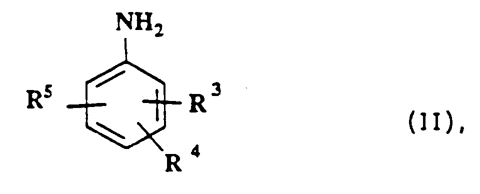 wobei R³, R⁴ und R⁵ Wasserstoffe, C₁-C₄-Alkylgruppen, Hydroxygruppen, Carboxylgruppen, Sulfogruppen, C₁-C₄-Amino-alkylgruppen oder NR⁶R⁷-Gruppen darstellen, wobei R⁶ und R⁷ unabhängig voneinander für Wasserstoffe, C₁-C₄-Alkylgruppen, Arylgruppen oder C₂-C₄-Hydroxyalkylgruppen stehen, mit der Maßgabe, daß höchstens zwei der Gruppen R³, R⁴, R⁵ gleichzeitig Wasserstoffe und/oder C₁-C₄-Alkylgruppen darstellen; wobei zwei der Gruppen R³, R⁴ und R⁵ gemeinsam auch einen ankondensierten Benzolring bilden können, der gegebenenfalls mit C₁-C₄-Alkyl, Hydroxy, Carboxyl, Sulfo, C₁-C₄-Aminoalkyl oder Amino substituiert ist.

Beispiele sind z. B. Sulfaninsäure, 1,5-, 1,8-, 2,3-Diaminonaphthalin, 6-Amino-1-naphthol-3-sulfonsäure, 4-Amino-, 4,4'-Diaminodiphenylamin, 4,4'-Diaminodiphenylamin-2-sulfonsäure.

Bevorzugt sind diejenigen Färbemittel, in denen das aromatische Amin der Formel II ausgewählt ist aus der Gruppe p-Toluylendiamin, 4-Aminophenol, 4-Amino-2-aminomethylphenol, 3,4-Diaminobenzoesäure, 2,4,5,6-Tetraaminobenzol, 1-(β-Hydroxyethyl)-2,5-diaminobezol.

Ein weiterer Erfindungsgegenstand sind Mittel zum Färben von keratinhaltigen Fasern enthaltend mindestens ein Isatinderivat der Formel I und mindestens ein aromatisches Amin der Formel III 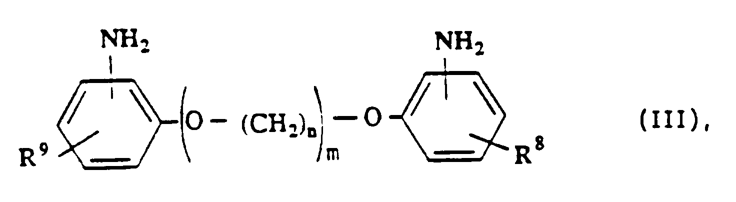 wobei R⁸ und R⁹ unabhängig voneinander Wasserstoffe, C₁-C₄-Alkylgruppen, Hydroxygruppen oder Aminogruppen darstellen, n eine ganze Zahl von 2 bis 4 und m eine ganze Zahl von 1 bis 4 ist.

Bevorzugt sind diejenigen Färbemittel, in denen das aromatische Amin der Formel III 1,3-Bis-(2,4-diaminophenoxy)propan ist.

Ein weiterer Erfindungsgegenstand sind Mittel zum Färben von keratinhaltigen Fasern enthaltend mindestens ein Isatinderivat der Formel I und mindestens ein Aminopyrimidin der Formel IV 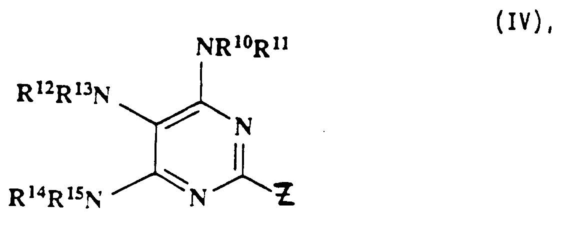 wobei R¹⁰, R¹¹, R¹², R¹³, R¹⁴ und R¹⁵ unabhängig voneinander Wasserstoffe, C₁-C₄-Alkylgruppen oder C₂-C₄-Hydroxyalkylgruppen darstellen und Z für Wasserstoff, eine OH- oder eine NR¹⁶R¹⁷-Gruppe steht, in der R¹⁶ und R¹⁷ unabhängig voneinander Wasserstoffe, C₁-C₄-Alkylgruppen oder C₂-C₄-Hydroxyalkylgruppen darstellen.

Bevorzugt sind dabei diejenigen Färbemittel, in denen unsubstituiertes Tetraaminopyrimidin eingesetzt wird.

Ein weiterer Erfindungsgegenstand sind Mittel zum Färben von keratinhaltigen Fasern enthaltend mindestens ein Isatinderivat der Formel I und
- mindestens ein Indolderivat der Formel Va 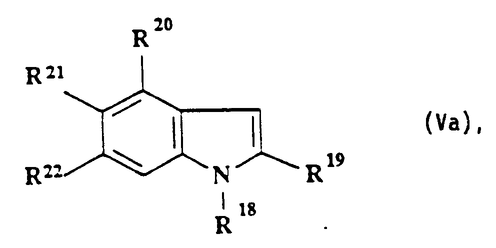 wobei R¹⁸ Wasserstoff, eine C₁-C₄-Alkyl- oder C₂-C₄-Acylgruppe, R¹⁹ Wasserstoff oder eine Carboxylgruppe bedeutet und R²⁰, R²¹ und R²² Wasserstoffe, C₁-C₄-Alkylgruppen, C₂-C₄-Acyloxygruppen, Hydroxygruppen, Aminogruppen oder C₁-C₄-Alkoxygruppen darstellen, mit der Maßgabe, daß höchstens zwei der Gruppen R²⁰, R²¹, R²² gleichzeitig Nasserstoffe und/oder C₁-C₄-Alkylgruppen darstellen, oder
- mindestens ein Indolinderivat der Formel Vb 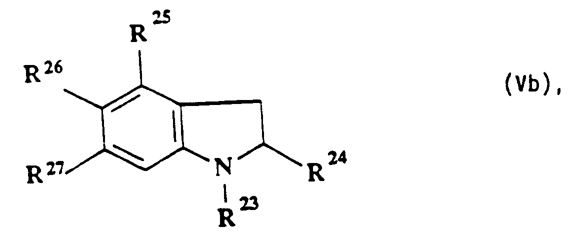 wobei R²³ Wasserstoff, eine C₁-C₄-Alkyl- oder C₂-C₄-Acylgruppe, R²⁴ Wasserstoff oder eine Carboxylgruppe bedeutet und R²⁵, R²⁶ und R²⁷ Wasserstoffe, C₁-C₄-Alkylgruppen, C₂-C₄-Acyloxygruppen, Hydroxygruppen, Aminogruppen oder C₁-C₄-Alkoxygruppen darstellen, mit der Maßgabe, daß höchstens zwei der Gruppen R²⁵, R²⁶, R²⁷ gleichzeitig Wasserstoffe und/oder C₁-C₄-Alkylgruppen darstellen.

Bevorzugt sind diejenigen Färbemittel, in denen das Indolderivat der Formel Va bzw. das Indolinderivat der Formel Vb ausgewählt ist aus der Gruppe 5,6-Dihydroxyindol, 5,6-Diacetoxyindol, 4-Hydroxyindolin, 6-Aminoindolin und 5,6-Dihydroxyindolin.

Alle in den erfindungsgemäßen Färbemitteln zum Einsatz kommenden Verbindungen mit substituierter oder unsubstituierter Aminofunktion können sowohl in freier Form als auch in Form ihrer wasserlöslichen Salze eingesetzt werden. Als wasserlösliche Salze sind ohne Einschränkung z.B. Hydrochloride, Hydrobromide oder Sulfate zu nennen.

In allen erfindungsgemäßen Färbemitteln können auch mehrere verschiedene Isatinderivate der Formel I gemeinsam zum Einsatz kommen; ebenso können auch mehrere verschiedene Aminosäuren bzw. Oligopeptide sowie mehrere verschiedene Verbindungen der Formeln II, III, IV, Va und Vb gemeinsam verwendet werden.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren.

Zur Anwendung auf dem menschlichen Haar können die erfindungsgemäßen Färbemittel in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf dem Haar geeignet sind. Die Isatine der Formel I sowie die Aminosäuren und Oligopeptide bzw. die Verbindungen der Formeln II, III, IV, Va und Vb sind dabei in einer Menge von jeweils 0,1 bis 20, vorzugsweise 1 bis 7 Gew.-%, jeweils bezogen auf die gesamte Färbemittelzubereitung, enthalten.

Der wasserhaltige kosmetische Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z.B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Der pH-Wert der gebrauchsfertigen Färbezubereitungen liegt zwischen 2 und 11, vorzugsweise zwischen 5 und 9. Bei Verwendung von luftoxidablen Komponenten wie z.B. 5,6-Dihydroxyindol, 5,6-Dihydroxyindolin und deren Derivaten ist ein schwach alkalischer pH-Wert von 7 - 11, vorzugsweise 8 - 10, vorteilhaft.

Zum Haarefärben werden die erfindungsgemäßen Färbemittel in Form des wasserhaltigen, kosmetischen Trägers in einer Menge von 100 g auf das Haar aufgebracht, ca. 30 Minuten dort belassen und dann ausgespült oder mit einem handelsüblichen Haarsphampoo ausgewaschen.

Wenn die erfindungsgemäßen Färbemittel in Form einer wasserhaltigen kosmetischen Zubereitung vorliegen, ist es vorteilhaft (aber nicht notwendig), das Isatinderivat der Formel I getrennt von der zweiten reaktiven Komponente, d.h. der Aminosäure oder dem Oligopeptid bzw. der Verbindungen der Formeln II, III, IV, Va und Vb, zu konfektionieren.

Die beiden Komponenten (Isatinderivat der Formel I und zweite reaktive Komponente) können dann entweder gleichzeitig auf das Haar aufgebracht werden oder aber auch nacheinander, wobei es keine Rolle spielt, welche der beiden Komponenten zuerst aufgetragen wird; zwischen dem Auftragen der ersten und der zweiten Komponente können bis zu 30 Minuten Zeitabstand liegen.

Besonders vorteilhaft ist es jedoch, alle Komponenten gemeinsam in einer wasserfreien, pulverförmigen Zubereitung zu konfektionieren.

Ein weiterer Erfindungsgegenstand sind deshalb Haarfärbemittel in Form eines Pulvers enthaltend mindestens ein Isatinderivat der Formel I in einer Menge von 1 bis 90 Gew.-%, vorzugsweise 20 bis 50 Gew.-%, und eine Aminosäure oder ein aus 2 bis 9 Aminosäuren aufgebautes Oligopeptid in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus Isatin-5-sulfonsäure, Isatin-4-carbonsäure und Isatin-5-carbonsäure und die Aminosäure bzw. das Oligopeptid ausgewählt sind aus Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

Ein weiterer Erfindungsgegenstand sind Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel II in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus Isatin-5-sulfonsäure, Isatin-4-carbonsäure und Isatin-5-carbonsäure und das aromatische Amin der Formel II ausgewählt ist aus der Gruppe p-Toluylendiamin, 4-Aminophenol, 4-Amino-2-aminomethylphenol, 3,4-Diaminobenzoesäure, 2,4,5,6-Tetraaminobenzol, 1-(β-Hydroxyethyl )-2,5-diaminobenzol.

Ein weiterer Erfindungsgegenstand sind Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel III in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus Isatin-5-sulfonsäure, Isatin-4-carbonsäure und Isatin-5-carbonsäure und das aromatische Amin der Formel III 1,3-Bis-(2,4-diaminophenoxy)-propan ist.

Ein weiterer Erfindungsgegenstand sind Haarfärbemittel in Form eines Pulvers enthaltend mindestens ein Isatinderivat der Formel I in einer Menge von 1 bis 90, vorzugsweise 20 bis 50 Gew.-%, und mindestens ein Aminopyrimidin der Formel IV in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus Isatin-5-sulfonsäure, Isatin-4-carbonsäure und Isatin-5-carbonsäure und das Aminopyrimidin der Formel IV unsubstituiertes Tetraaminopyrimidin ist.

Ein weiterer Erfindungsgegenstand sind Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein Indolderivat der Formel Va oder ein Indolinderivat der Formel Vb in einer Menge von 1 bis 90 Gew.-%, vorzugsweise 20 bis 50 Gew.-%, jeweils bezogen auf das gesamte Pulverfärbemittel.

Besonders bevorzugt sind dabei solche Haarfärbemittel, in denen das Isatinderivat der Formel I ausgewählt ist aus Isatin-5-sulfonsäure, Isatin-4-carbonsäure und Isatin-5-carbonsäure und das Indolderivat der Formel Va bzw. das Indolinderivat der Formel Vb ausgewählt sind aus 5,6-Dihydroxyindol, 5,6-Diacetoxyindol, 4-Hydroxyindolin, 6-Aminoindolin und 5,6-Dihydroxyindolin.

Die Isatine der Formel I können vorteilhafterweise auch mit Pyridinen wie z. B. 2,6-Dihydroxy-3,4-dimethyl-, 2-Amino-3-hydroxy-, 2-Methylamino-3-amino-6-methoxy-, 3,5-Diamino-2,6-dimethoxypyridin als zweiter Komponente kombiniert werden.

Im einfachsten Falle enthalten die pulverförmigen Haarfärbemittel außer den beiden reaktiven Komponenten (Isatinderivat der Formel I und Aminosäure oder Oligopeptid bzw. Verbindungen der Formeln II, III, IV, Va und Vb) lediglich ein wasserlösliches polymeres Verdickungsmittel. Das Verdickungsmittel hat die Aufgabe, der nach Zugabe von Wasser erhaltenen gebrauchsfertigen Haarfärbemittelzubereitung die anwendungstechnisch gewünschte Konsistenz zu verleihen. Geeignete Verdickungsmittel sind z.B. natürliche Gummen, z. B. Guarkernmehl, Gummi arabicum, Karaya-Gummi, Johannisbrotkernmehl, Leinsamengummen und Pektin, biosynthetische Gummen wie z.B. Xanthan-Gummi und Dextrane, synthetische Gummen wie z.B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose.

Weitere fakultative Komponenten in dem Pulverhaarfärbemittel sind Metallsalze, z.B. die Acetate, Sulfate, Chloride, Bromide, Carbonate, Glykolate, Lactate oder Gluconate der Alkali- und Erdalkalimetalle, weiterhin haarpflegende Zusätze wie z.B. wasserlösliche kationische Polymere, anionische Polymere, amphotere oder zwitterionische Polymere, Pantothensäure, Vitamine, Pflanzenextrakte, Komplexbildner, Parfümkomponenten sowie Reduktionsmittel wie z.B. Ascorbinsäure.

Zur Bereitung der gebrauchsfertigen Haarfärbemittel-Zubereitung werden 1 bis 30 g, vorzugsweise 3 bis 15 g des Pulvers mit heißem Wasser von 90 bis 100°C auf 100 g aufgefüllt.Zum Lösen des Pulvers können auch Wasser/Alkohol-Gemische oder andere kosmetisch verträgliche Lösungsmittel verwendet werden.

Nach Abkühlen auf ca. 40°C wird die gebrauchsfertige Haarfärbemittel-Zubereitung auf das Haar aufgebracht und dort 30 Minuten lang belassen. Anschließend wird das Haar gespült oder mit einem handelsüblichen Haarshampoo gewaschen.

Die folgenden Beispiele sollen den Erfindungsgegenstand näher erläutern, ohne ihn jedoch hierauf zu beschränken.

### Beispiele

### Beispiel I

Es wurde eine Haarfärbe-Emulsion der folgenden Zusammensetzung zubereitet:

| | |
|---|---|
| C₁₆₁₈-Fettalkohol | 8,5 g |
| C₁₂/₁₄-Fettalkoholethersulfat (28 %ig), Na-salz | 25 g |
| Isatin-5-sulfonsäure | 1 g |
| Wasser | ad 100 g |
| NH₃ | bis pH 9,0 |

Die Haarfärbe-Emulsion wurde auf ca. 5 cm lange Strähnen standardisierten, zu 90 % ergrauten Menschenhaars aufgetragen und dort 30 Minuten lang bei 32°C belassen. Nach Beendigung des Färbeprozesses wurde das Haar gespült, mit einem üblichen Haarsphampoo gewaschen und getrocknet. Es resultierte ein strohgelbe Nuance mit einer Lichtechtheit von 3.0 nach DIN 54004.

Eine Haarfärbe-Emulsion, bei der anstelle von Isatin-5-sulfonsäure Isatin verwendet wurde, ergab unter gleichen Bedingungen eine Nuance mit einer Lichtechtheit von nur 2.0 nach DIN 54004.

[DIN 54004 steht für "Deutsche Industrie-Norm 54004", Prüfung der Farbechtheit von Textilien, Bestimmung der Lichtechtheit von Färbungen und Drucken mit künstlichem Tageslicht, Ausgabe April 1966. Der Alleinverkauf der Normblätter erfolgt durch Beuth-Vertrieb GmbH, Berlin 30 und Köln, Deutschland. In dem Normblatt werden Meßanordnung und Meßdurchführung explizit beschrieben.]

### Beispiel II

Es wurden Pulverhaarfärbemittel der Zusammensetzungen 1 bis 12 zubereitet (Tabelle 1: Zahlenangaben sind in g).

| *Beispiele* 1 - 12 | | | | | | | | | | | | |
|---|---|---|---|---|---|---|---|---|---|---|---|---|
| *Rezeptur Nr.* | 1 | 2 | 3 | 4 | 5 | 6 | 7 | 8 | 9 | 10 | 11 | 12 |
| *Guarkernmehl (Emulkoll 35) Lucas Meyer)* | *1.0* | *1,0* | *1.0* | *1.0* | *1.0* | *1.0* | *1.0* | *1.0* | *1.0* | *1.0* | *1.0* | *1.0* |
| *Natriumacetat 3H20* | *1.4* | *1,4* | *1.4* | *1.4* | *1.4* | *1.4* | *1.4* | *1.4* | *1.4* | *1.4* | *1.4* | *1.4* |
| *Isatin-5-sulfonsäure, NaSalz* | *2.5* | *2.5* | *2.5* | *2.5* | *2.5* | *2.5* | *2.5* | *2.5* | *2.5* | *-* | *-* | *2.5* |
| *Isatin-4-carbonsäure* | *-* | *-* | *-* | *-* | *-* | *-* | *-* | *-* | *-* | *1,9* | *1,9* | *-* |
| *2,5-Diaminotoluolsulfat* | *2.2* | *-* | *-* | *-* | *-* | *-* | *-* | *2.2* | *-* | *-* | - | *-* |
| *1-(β-Hydroxethyl)-2,5-diamino-benzolsulfat* | *-* | *2.5* | *-* | *-* | *-* | *-* | *-* | *-* | *2.5* | *-* | *-* | *-* |
| *Tetraaminopyrimidinsulfat* | *-* | *-* | *2.6* | *-* | *-* | *-* | *-* | *-* | *-* | *-* | *-* | *-* |
| *4-Aminophenolhydrochlorid* | *-* | *-* | *-* | *1.4* | *-* | *-* | *-* | *-* | *-* | *-* | *-* | *-* |
| *4-Amino-2-aminomethylphenolhydrochlorid* | *-* | *-* | *-* | *-* | *2.1* | *-* | *-* | *2.1* | *-* | *-* | *2.1* | *-* |
| *3.4-Diaminobenzoesäure* | *-* | *-* | *-* | *-* | *-* | *1.5* | *-* | *-* | *-* | *-* | *-* | *-* |
| *1.3-Bis-(2.4-diaminophenoxy)propantetrahydrochlorid* | *-* | *-* | *-* | *-* | *-* | *-* | *4.4* | | | | | *-* |
| *5,6-Dihydroxyindolinhydrobromid* | *-* | *-* | *-* | *-* | *-* | *-* | *-* | *-* | *2.3* | *-* | *-* | *-* |
| *2,4,5,6, Tetraaminobenzol tetrahydrochlorid* | *-* | *-* | *-* | *-* | *-* | *-* | *-* | *-* | *-* | *2.8* | *-* | *-* |
| *L-Arginin* | *-* | *-* | *-* | *-* | *-* | *-* | *-* | *-* | *-* | *-* | *-* | *1,8* |

Zur Bereitung von gebrauchsfertigen Haarfärbezubereitungen wurden die Zusammensetzungen 1 bis 12 jeweils mit 90 bis 100°C heißem Wasser auf 100 g aufgefüllt und der pH-Wert mit Na₂CO₃ auf 9,0 eingestellt. Nach Abkühlen auf ca. 40°C wurden die gebrauchsfertigen Zubereitungen auf Strähnen aus hellblondem Naturhaar aufgebracht. Die Strähnen wurden dann in Aluminiumfolie verpackt und bei 32°C 30 Minuten lang im Trockenschrank belassen. Anschließend wurden sie shampooniert, ausgewachen und getrockent.

Die erzielten Farbnuancen sind Tabelle 2 zu entnehmen:

**Tabelle 2**

| Beispiel Nr.: | Farbe: |
|---|---|
| 1 | kirschrot |
| 2 | kirschrot |
| 3 | zinnoberrot |
| 4 | gelbliches Kupfer |
| 5 | gelbliches Kuper |
| 6 | neutrales Gelb |
| 7 | mittelblond |
| 8 | rötliches Kupfer |
| 9 | rötliches Braun |
| 10 | violettrot |
| 11 | rotbraun |
| 12 | pinkfarben |

Die Ausfärbungen zeigen eine hohe Waschechtheit.

### Beispiel III

Es wurde ein Haarfärbe-Gel der folgenden Zusammensetzung A zubereitet:

| | |
|---|---|
| Guarkernmehl | 1,0 g |
| 5,6-Diacetoxyindol | 2,3 g |
| Natriumacetat x 3H₂O | 1,4 g |
| Wasser | ad 100 g |
| Na₂CO₃ | bis pH = 9,0 |

Dieses Haarfärbe-Gel wurd auf Strähnen aus hellblondem Naturhaar aufgebracht. Die Strähnen wurden 30 Minuten lang bei 32°C im Trockenschrank belassen, anschließend wurden sie shampooniert und ausgewaschen. Dann wurde ein gebrauchsfertiges Haarfärbegel der Zusammensetzung Nr. 1 auf die Strähnen aufgebracht, die Strähne in Aluminiumfolie verpackt und 30 Minuten bei 32°C im Trockenschrank belassen. Danach wurden die Strähnen shampooniert, ausgewaschen und getrocknet. Es resultierte eine braunrote Nuance.

In einem Zweiten Färbeversuch wurde ein gebrauchsfertiges Haarfärbe-Gel der Zusammensetzung Nr. 4 auf hellblonde Naturhaar-Strähnen aufgebracht, die Strähnen in Aluminiumfolie verpackt, 30 Minuten lang bei 32°C im Trockenschrank belassen, shampooniert und ausgewaschen. Dann wurde das o.g. Haarfärbe-Gel der Zusammensetzung A aufgetragen, die Strähnen wurden 30 Minuten lang bei 32°C im Trockenschrank belassen, shampooniert, ausgewaschen und getrocknet. Es resultierte eine dunkel-kupferfarbene Nuance.

### Beispiel IV

Es wurde eine Aufschlämmung von 10 mMol eines Isatins der Formel I und 10 mMol einer zweiten Komponente in 100 ml Wasser bereitet. Die Aufschlämmung wurde auf Siedetemperatur erhitzt und nach dem Abkühlen filtriert, der pH-Wert wurde anschließend auf 6 eingestellt.
In diese Färbelösung wurden bei 35°C bzw. 23°C 30 Minuten lang zu 90 % ergraute, nicht vorbehandelte Menschenhaare eingebracht. Die jeweiligen Färbetemperaturen, Färbedauern, Farbnuancen und Farbtiefen sind Tabelle 3 zu entnehmen.

### Die Farbtiefe wurde dabei nach folgender Skala bewertet:

- -: : keine oder eine sehr blasse Ausfärbung
- (+): : schwache Intensität
- +: : mittlere Intensität
- +(+): : mittlere bis starke Intensität
- ++: : starke Intensität
- ++(+): : starke bis sehr starke Intensität
- +++: : sehr starke Intensität

**Tabelle 3**

| **Ausfärbungen mit Isatin-5-sulfonsäure, Na-salz** | | | |
|---|---|---|---|
| Zweite Komponente | Färbenuance | Färbetemperatur | Farbtiefe |
| 2,3-Diaminonaphthalin | gelb | 35°C | ++(+) |
| 1,8-Diaminonaphthalin | hellbraun | 35°C | +(+) |
| 1,5-Diaminonaphthalin | mittelbraun | 35°C | ++ |
| 6-Amino-1-naphthol-3-sulfonsäure | hellbraun | 35°C | ++ |
| N-phenyl-p-phenylendiamin x HCl | rotviolett | 35°C | +++ |
| 4,4'-Diaminodiphenylamin x H₂SO₄ | blauviolett | 35°C | +++ |
| 4,4'-Diaminodiphenylamin-2-sulfonsäure | rotviolett | 35°C | +(+) |
| 2,5-Diaminotoluol x H₂SO₄ | violettbraun | 23°C | ++ |
| 2,4,5,6-Tetraaminopyrimidin x H₂SO₄ | violettrot | 23°C | ++ |
| 3,4-Diaminobenzoesäure | gelbblond | 23°C | ++ |
| 4-(4-Amino-m-toluidino)-phenol | dunkelviolett | 30°C | +++ |
| 3,5-Diamino-2,6-dimethoxypyridin | dunkelgraublau | 30°C | ++(+) |

## Patentansprüche

1. Verwendung von Isatinderivaten der Formel I 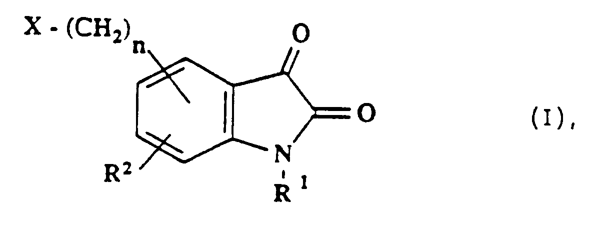 wobei R¹ Wasserstoff, eine C₁-C₄-Alkyl-, C₂-C₄-Hydroxyalkyl-, C₂-C₂₀-Acyl-, Benzyl- oder Phenylgruppe, R² Wasserstoff, eine C₁-C₄-Alkyl-, C₁-C₄-Alkoxy, Hydroxy-, Halogen oder Nitrogruppe, X eine Sulfogruppe - SO₃H oder eine Carboxylgruppe - COOH darstellt und n eine ganze Zahl von 0 bis 3 ist, und deren wasserlöslichen Salzen zum Färben von keratinhaltigen Fasern.

2. Verwendung von Isatinderivaten der Formel I nach Anspruch 1, dadurch gekennzeichnet, daß R¹ und R² Wasserstoffe darstellen und n gleich 0 ist.

3. Verwendung von Isatin-5-sulfonsäure, Isatin-4-carbonsäure und Isatin-5-carbonsäure nach Anspruch 1 und 2.

4. Mittel zum Färben von keratinhaltigen Fasern, enthaltend mindestens ein Isatinderivat der Formel I und mindestens eine Aminosäure oder ein aus 2 bis 9 Aminosäuren aufgebautes Oligopeptid.

5. Mittel nach Anspruch 4, dadurch gekennzeichnet, daß die Aminosäure oder das Oligopeptid ausgewählt sind aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

6. Mittel zum Färben von keratinhaltigen Fasern enthaltend mindestens ein Isatinverdivat der Formel I und mindestens ein aromatisches Amin der Formel II 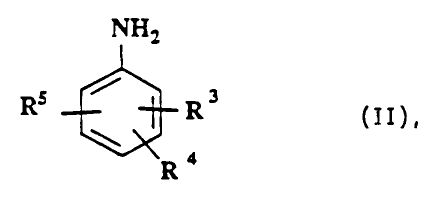 wobei R³, R⁴ und R⁵ Wasserstoffe, C₁-C₄-Alkylgruppen, Hydroxygruppen, Carboxylgruppen, Sulfogruppen, C₁-C₄-Aminoalkylgruppen oder NR⁶R⁷-Gruppen darstellen, wobei R⁶ und R⁷ unabhängig voneinander für Wasserstoffe, C₁-C₄-Alkylgruppen, Arylgruppen, oder C₂-C₄-Hydroxyalkylgruppen stehen, mit der Maßgabe, daß höchstens zwei der Gruppen R³, R⁴, R⁵ gleichzeitig Wasserstoffe und/oder C₁-C₄-Alkylgruppen darstellen;
wobei zwei der Gruppen R³, R⁴ und R⁵ gemeinsam auch einen ankondensierten Benzolring bilden können, der gegebenenfalls mit C₁-C₄-Alkyl, Hydroxy, Carboxyl, Sulfo, C₁-C₄-Aminoalkyl oder Amino substituiert ist.

7. Mittel nach Anspruch 6, dadurch gekennzeichnet, daß das aromatische Amin der Formel II ausgewählt ist aus der Gruppe p-Toluylendiamin, 4-Aminophenol, 4-Amino-2-aminomethylphenol, 3,4-Diaminobenzoesäure, 2,4,5,6-Tetraaminobenzol, 1-(β-Hydroxyethyl)-2,5-diaminobozol.

8. Mittel zum Färben von keratinhaltigen Fasern enthaltend mindestens ein Isatinderivat der Formel I und mindestens ein aromatisches Amin der Formel III 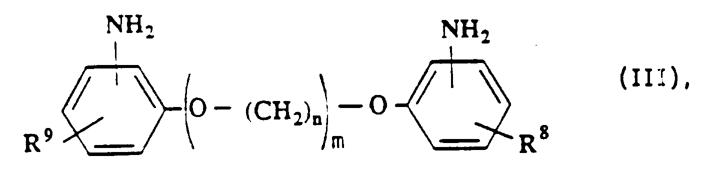 wobei R⁸ und R⁹ unabhängig voneinander Wasserstoffe, C₁-C₄-Alkylgruppen, Hydroxygruppen oder Aminogruppen darstellen, n eine ganze Zahl von 2 bis 4 ist und m eine ganze Zahl von 1 bis 4 ist.

9. Mittel nach Anspruch 8, dadurch gekennzeichnet, daß das aromatische Amin der Formel III 1,3-Bis-(2,4-diaminophenoxy)propan ist.

10. Mittel zum Färben von keratinhaltigen Fasern enthaltend mindestens ein Isatinderivat der Formel I und mindestens ein Aminopyrimidin der Formel IV 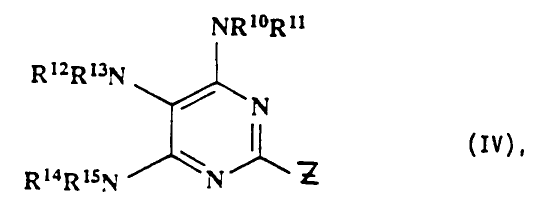 wobei R¹⁰, R¹¹, R¹², R¹³, R¹⁴ und R¹⁵ unabhängig voneinander Wasserstoffe, C₁-C₄-Alkylgruppen oder C₂-C₄-Hydroxyalkylgruppen darstellen und Z für Wasserstoff, eine OH- oder eine NR¹⁶R¹⁷-Gruppe steht, in der R¹⁶ und R¹⁷ unabhängig voneinander Wasserstoffe, C₁-C₄-Alkylgruppen oder C₂-C₄-Hydroxyalkylgruppen darstellen.

11. Mittel nach Anspruch 10, dadurch gekennzeichnet, daß in dem Tetraaminopyrimidin der Formel IV die Gruppen R¹⁰ bis R¹⁷ Wasserstoffe darstellen.

12. Mittel zum Färben von keratinhaltigen Fasern enthaltend mindestens ein Isatinderivat der Formel I und
- mindestens ein Indolderivat der Formel Va 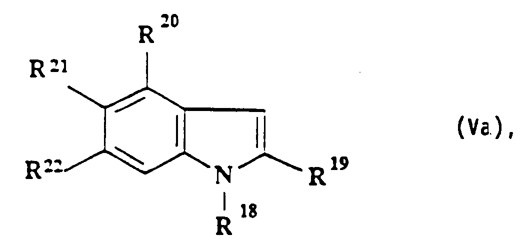 wobei R¹⁸ Wasserstoff, eine C₁-C₄-Alkyl- oder C₂-C₄-Acylgruppe, R¹⁹ Wasserstoff oder eine Carboxylgruppe bedeutet und R²⁰, R²¹ und R²² Wasserstoffe, C₁-C₄-Alkylgruppen, C₂-C₄-Acyloxygruppen, Hydroxygruppen, Aminogruppen oder C₁-C₄-Alkoxygruppen darstellen, mit der Maßgabe, daß höchstens zwei der Gruppen R²⁰, R²¹, R²² gleichzeitig Wasserstoffe und/oder C₁-C₄-Alkylgruppen darstellen, oder
- mindestens ein Indolinderivat der Formel Vb 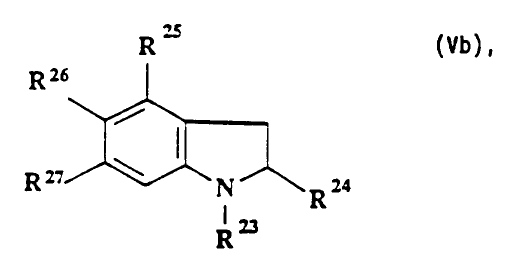 wobei R²³ Wasserstoff, eine C₁-C₄-Alkyl- oder C₂-C₄-Acylgruppe, R²⁴ Wasserstoff oder eine Carboxylgruppe bedeutet und R²⁵, R²⁶ und R²⁷ Wasserstoffe, C₁-C₄-Alkylgruppen, C₂-C₄-Acyloxygruppen, Hydroxygruppen, Aminogruppen oder C₁-C₄-Alkoxygruppen darstellen, mit der Maßgabe, daß höchstens zwei der Gruppen R²⁵, R²⁶, R²⁷ gleichzeitig Wasserstoffe und/oder C₁-C₄Alkylgruppen darstellen.

13. Mittel nach Anspruch 12, dadurch gekennzeichnet, daß das Indolderivat der Formel Va bzw. das Indolinderivat der Formel Vb ausgewählt ist aus der Gruppe 5,6-Dihydroxyindol, 5,6-Diacetoxyindol, 4-Hydroxyindolin, 6-Aminoindolin und 5,6-Dihydroxyindolin.

14. Haarfärbemittel in Form eines Pulvers enthaltend mindestens ein Isatinderivat der Formel I in einer Menge von 1 bis 90 Gew.-%, vorzugsweise 20 bis 50 Gew.-%, und eine Aminosäure oder ein aus 2 bis 9 Aminosäuren aufgebautes Oligopeptid in einer Menge von 1 bis 90 Gew.-%, vorzugsweise 20 bis 50 Gew.-%, jeweils bezogen auf das gesamte Pulverfärbemittel.

15. Haarfärbemittel nach Anspruch 14, dadurch gekennzeichnet, daß das Isatinderivat der Formel I ausgewählt ist aus Isatin-5-sulfonsäure, Isatin-4-carbonsäure und Isatin-5-carbonsäure und die Aminosäure bzw. das Oligopeptid ausgewählt sind aus Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, 3,4-Dihydroxyphenylalanin, Serin, Ornithin, Threonin, Glutathion.

16. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel II in einer Menge von 1 bis 90 Gew.-%, vorzugsweise 20 bis 50 Gew.-%, jeweils bezogen auf das gesamte Pulverfärbemittel.

17. Haarfärbemittel nach Anspruch 16, dadurch gekennzeichnet, daß das Isatinderivat der Formel I ausgewählt ist aus Isatin-5-sulfonsäure, Isatin-4-carbonsäure und Isatin-5-carbonsäure und das aromatische Amin der Formel II ausgewählt ist aus p-Toluylendiamin, 4-Aminophenol, 4-Amino-2-aminomethylphenol, 3,4-Diaminobenzoesäure, 2,4,5,6-Tetraaminobenzol, 1-(β-Hydroxyethyl)-2,5-diaminobezol.

18. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein aromatisches Amin der Formel III in einer Menge von 1 bis 90 Gew.-%, vorzugsweise 20 bis 50 Gew.-%, jeweils bezogen auf das gesamte Pulverfärbemittel.

19. Haarfärbemittel nach Anspruch 18, dadurch gekennzeichnet, daß das Isatinderivat der Formel I ausgewählt ist aus Isatin-5-sulfonsäure, Isatin-4-carbonsäure und Isatin-5-carbonsäure und das aromatische Amin der Formel III 1,3-Bis-(2,4-diaminophenoxy)-propan ist.

20. Haarfärbemittel in Form eines Pulvers enthaltend mindestens ein Isatinderivat der Formel I in einer Menge von 1 bis 90, vorzugsweise 20 bis 50 Gew.-%, und mindestens ein Aminopyrimidin der Formel IV in einer Menge von 1 bis 90 Gew.-%, vorzugsweise 20 bis 50 Gew.-%, jeweils bezogen auf das gesamte Pulverfärbemittel.

21. Haarfärbemittel nach Anspruch 20, dadurch gekennzeichnet, daß das Isatinderivat der Formel I ausgewählt ist aus Isatin-5-sulfonsäure, Isatin-4-carbonsäure und Isatin-5-carbonsäure und das Aminopyrimidin der Formel IV unsubstituiertes Tetraaminopyrimidin ist.

22. Haarfärbemittel in Form eines Pulvers enthaltend mindestens ein Isatinderivat der Formel I in einer Menge von 1 bis 90 Gew.-%, vorzugsweise 20 bis 50 Gew.-%, und mindestens ein Indolderivat der Formel Va oder ein Indolinderivat der Formel Vb in einer Menge von 1 bis 90 Gew.-%, vorzugsweise 20 bis 50 Gew.-%, jeweils bezogen auf das gesamte Pulverfärbemittel.

23. Haarfärbemittel nach Anspruch 22, dadurch gekennzeichnet, daß das Isatinderivat der Formel I ausgewählt ist aus Isatin-5-sulfonsäure, Isatin-4-carbonsäure und Isatin-5-carbonsäure und das Indolderivat der Formel Va bzw. das Indolinderivat der Formel Vb ausgewählt sind aus 5,6-Dihydroxyindol, 5,6-Diacetoxyindol, 4-Hydroxyindolin, 6-Aminoindolin und 5,6-Dihydroxyindolin.

## Claims

1. The use of isatin derivatives corresponding to formula I: 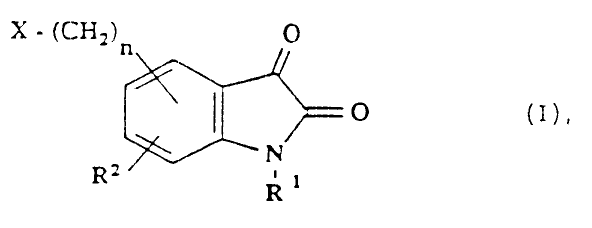 in which R¹ is hydrogen, a C₁₋₄ alkyl, C₂₋₄ hydroxyalkyl, C₂₋₂₀ acyl, benzyl or phenyl group, R² is hydrogen, a C₁₋₄ alkyl, C₁₋₄ alkoxy, hydroxy, halogen or nitro group, X is a sulfo group -SO₃H or a carboxyl group -COOH and n is an integer of 0 to 3,
and water-soluble salts thereof for coloring keratin-containing fibers.

2. The use of isatin derivatives corresponding to formula I as claimed in claim 1, characterized in that R¹ and R² are hydrogens and n = 0.

3. The use of isatin-5-sulfonic acid, isatin-4-carboxylic acid and isatin-5-carboxylic acid as claimed in claims 1 and 2.

4. Formulations for coloring keratin-containing fibers containing at least one isatin derivative corresponding to formula I and at least one amino acid or an oligopeptide made up of 2 to 9 amino acids.

5. A formulation as claimed in claim 4, characterized in that the amino acid or the oligopeptide is selected from the group consisting of arginine, cysteine, methionine, proline, tyrosine, valine, glycine, glutamic acid, histidine, aspartic acid, alanine, tryptophan, cystine, lysine, hydroxyproline, leucine, isoleucine, phenyl alanine, 3,4-dihydroxyphenyl alanine, serine, ornithine, threonine and glutathione.

6. Formulations for coloring keratin-containing fibers containing at least one isatin derivative corresponding to formula I and at least one aromatic amine corresponding to formula II: 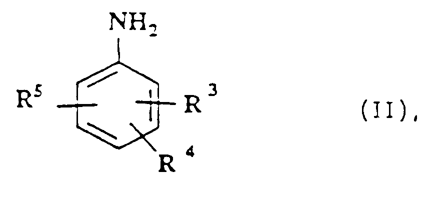 in which R³, R⁴ and R⁵ are hydrogens, C₁₋₄ alkyl groups, hydroxy groups, carboxyl groups, sulfo groups, C₁₋₄ aminoalkyl groups or NR⁶R⁷ groups, where R⁶ and R⁷ independently of one another are hydrogens, C₁₋₄ alkyl groups, aryl groups or C₂₋₄ hydroxyalkyl groups, with the proviso that at most two of the groups R³, R⁴ and R⁵ are simultaneously hydrogens and/or C₁₋₄ alkyl groups; two of the groups R³, R⁴ and R⁵ together may also form a fused benzene ring optionally substituted by C₁₋₄ alkyl, hydroxy, carboxyl, sulfo, C₁₋₄ aminoalkyl or amino.

7. Formulations as claimed in claim 6, characterized in that the aromatic amine corresponding to formula II is selected from the group consisting of p-tolylenediamine, 4-aminophenol, 4-amino-2-aminomethylphenol, 3,4-diaminobenzoic acid, 2,4,5,6-tetraaminobenzene, 1-(β-hydroxyethyl)-2,5-diaminobenzene.

8. Formulations for coloring keratin-containing fibers containing at least one isatin derivative corresponding to formula I and at least one aromatic amine corresponding to formula III: 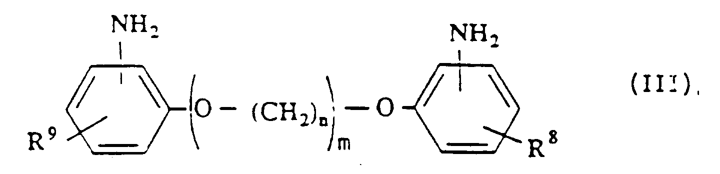 in which R⁸ and R⁹ independently of one another represent hydrogens, C₁₋₄ alkyl groups, hydroxy groups or amino groups, n is an integer of 2 to 4 and m is an integer of 1 to 4.

9. Formulations as claimed in claim 8, characterized in that the aromatic amine corresponding to formula III is 1,3-bis-(2,4-diaminophenoxy)-propane.

10. Formulations for coloring keratin-containing fibers containing at least one isatin derivative corresponding to formula I and at least one aminopyrimidine corresponding to formula IV: 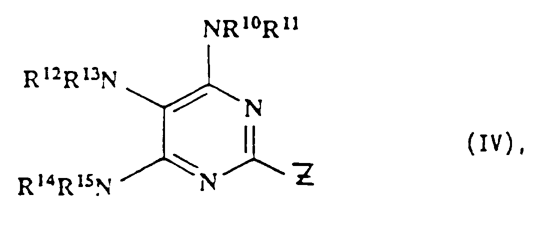 in which R¹⁰, R¹¹, R¹², R¹³, R¹⁴ and R¹⁵ independently of one another are hydrogens, C₁₋₄ alkyl groups or C₂₋₄ hydroxyalkyl groups and Z is hydrogen, an OH group or an NR¹⁶R¹⁷ group, where R¹⁶ and R¹⁷ independently of one another are hydrogens, C₁₋₄ alkyl groups or C₂₋₄ hydroxyalkyl groups.

11. Formulations as claimed in claim 10, characterized in that the substituents R¹⁰ to R¹⁷ in the tetraaminopyrimidine corresponding to formula IV are hydrogens.

12. Formulations for coloring keratin-containing fibers containing at least one isatin derivative corresponding to formula I and
- at least one indole derivative corresponding to formula Va: 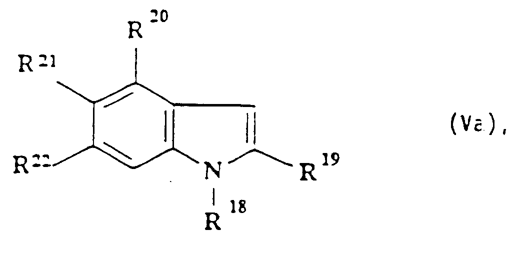 in which R¹⁸ is hydrogen, a C₁₋₄ alkyl or C₂₋₄ acyl group, R¹⁹ is hydrogen or a carboxyl group and R²⁰, R²¹ and R²² are hydrogens, C₁₋₄ alkyl groups, C₂₋₄ acyloxy groups, hydroxy groups, amino groups or C₁₋₄ alkoxy groups, with the proviso that at most two of the groups R²⁰, R²¹ and R²² are simultaneously hydrogens and/or C₁₋₄ alkyl groups, or
- at least one indoline derivative corresponding to formula Vb: 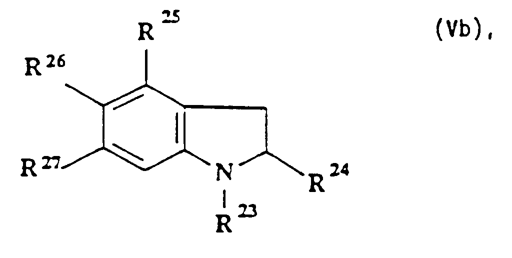 in which R²³ is hydrogen, a C₁₋₄ alkyl or C₂₋₄ acyl group, R²⁴ is hydrogen or a carboxyl group and R²⁵, R²⁶ and R²⁷ are hydrogens, C₁₋₄ alkyl groups, C₂₋₄ acyloxy groups, hydroxy groups, amino groups or C₁₋₄ alkoxy groups, with the proviso that at most two of the groups R²⁵, R²⁶ and R²⁷ are simultaneously hydrogens and/or C₁₋₄ alkyl groups.

13. Formulations as claimed in claim 12, characterized in that the indole derivative corresponding to formula Va or the indoline derivative corresponding to formula Vb is selected from the group consisting of 5,6-dihydroxyindole, 5,6-diacetoxyindole, 4-hydroxyindoline, 6-aminoindoline and 5,6-dihydroxyindoline.

14. Hair coloring formulations in the form of a powder containing at least one isatin derivative corresponding to formula I in a quantity of 1 to 90% by weight and preferably 20 to 50% by weight and an amino acid or an oligopeptide made up of 2 to 9 amino acids in a quantity of 1 to 90% by weight and preferably 20 to 50% by weight, based on the powder-form coloring formulation as a whole.

15. Hair coloring formulations as claimed in claim 14, characterized in that the isatin derivative corresponding to formula I is selected from isatin-5-sulfonic acid, isatin-4-carboxylic acid and isatin-5-carboxylic acid and the amino acid or the oligopeptide is selected from arginine, cysteine, methionine, proline, tyrosine, valine, glycine, glutamic acid, histidine, aspartic acid, alanine, tryptophan, cystine, lysine, hydroxyproline, leucine, isoleucine, phenyl alanine, 3,4-dihydroxyphenyl alanine, serine, ornithine, threonine and glutathione.

16. Hair coloring formulations in the form of a powder containing at least one isatin derivative corresponding to formula I in a quantity of 1 to 90% by weight and preferably 20 to 50% by weight and at least one aromatic amine corresponding to formula II in a quantity of 1 to 90% by weight and preferably 20 to 50% by weight, based on the powder-form coloring formulation as a whole.

17. Hair coloring formulations as claimed in claim 16, characterized in that the isatin derivative corresponding to formula I is selected from isatin-5-sulfonic acid, isatin-4-carboxylic acid and isatin-5-carboxylic acid and the aromatic amine corresponding to formula II is selected from the group consisting of p-tolylenediamine, 4-aminophenol, 4-amino-2-aminomethylphenol, 3,4-diaminobenzoicacid, 2,4,5,6-tetraaminobenzene, 1-(β-hydroxyethyl)-2,5-diaminobenzene.

18. Hair coloring formulations in the form of a powder containing at least one isatin derivative corresponding to formula I in a quantity of 1 to 90% by weight and preferably 20 to 50% by weight and at least one aromatic amine corresponding to formula III in a quantity of 1 to 90% by weight and preferably 20 to 50% by weight, based on the powder-form coloring formulation as a whole.

19. Hair coloring formulations as claimed in claim 18, characterized in that the isatin derivative corresponding to formula I is selected from isatin-5-sulfonic acid, isatin-4-carboxylic acid and isatin-5-carboxylic acid and the aromatic amine corresponding to formula III is 1,3-bis-(2,4-diaminophenoxy)-propane.

20. Hair coloring formulations in the form of a powder containing at least one isatin derivative corresponding to formula I in a quantity of 1 to 90% by weight and preferably 20 to 50% by weight and at least one aminopyrimidine corresponding to formula IV in a quantity of 1 to 90% by weight and preferably 20 to 50% by weight, based on the powder-form coloring formulation as a whole.

21. Hair coloring formulations as claimed in claim 20, characterized in that the isatin derivative corresponding to formula I is selected from isatin-5-sulfonic acid, isatin-4-carboxylic acid and isatin-5-carboxylic acid and the aminopyrimidine corresponding to formula IV is unsubstituted tetraaminopyrimidine.

22. Hair coloring formulations in the form of a powder containing at least one isatin derivative corresponding to formula I in a quantity of 1 to 90% by weight and preferably 20 to 50% by weight and at least one indole derivative corresponding to formula Va or an indoline derivative corresponding to formula Vb in a quantity of 1 to 90% by weight and preferably 20 to 50% by weight, based on the powder-form coloring formulation as a whole.

23. Hair coloring formulations as claimed in claim 22, characterized in that the isatin derivative corresponding to formula I is selected from isatin-5-sulfonic acid, isatin-4-carboxylic acid and isatin-5-carboxylic acid and the indole derivative corresponding to formula Va and the indoline derivative corresponding to formula Vb are selected from 5,6-dihydroxyindole, 5,6-diacetoxyindole, 4-hydroxyindoline, 6-aminoindoline and 5,6-dihydroxyindoline.

## Revendications

1. Utilisation de dérivés d'isatine de formule I 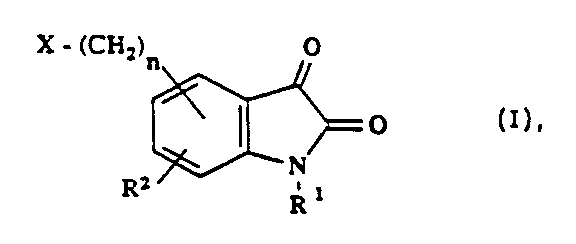 où R¹ représente un hydrogène, un groupe alkyle en C₁-C₄, hydroxyalkyle en C₂-C₄, acyle en C₂-C₂₀, benzyle en C₂-C₂₀ ou phényle en C₂-C₂₀, R² représente un hydrogène, un groupe alkyle en C₁-C₄, un groupe alcoxy en C₁-C₄, hydroxy, halogène ou nitro, X représente un groupe sulfo -SO₃H ou un groupe carboxyle - COOH et n est un nombre entier compris entre 0 et 3, et leurs sels hydrosolubles pour teindre des fibres contenant de la kératine.

2. Utilisation de dérivés d'isatine de formule I selon la revendication 1,
caractérisée en ce que
R¹ et R² représentent des atomes d'hydrogène et n est égal à 0.

3. Utilisation d'acide isatine-5-sulfonique, d'acide isatine-4-carboxylique et d'acide isatine-5-carboxylique selon les revendications 1 et 2.

4. Agents pour teindre des fibres contenant de la kératine, contenant au moins un dérivé d'isatine de formule I et au moins un aminoacide ou un oligopeptide constitué de 2 à 9 aminoacides.

5. Agents selon la revendication 4,
caractérisés en ce qu'
on choisit l'aminoacide ou l'oligopeptide dans le groupe des : arginine, cystéine, méthionine, proline, tyrosine, valine, glycine, acide glutamique, histidine, acide asparagique, alanine, tryptophane, cystine, lysine, hydroxyproline, leucine, Isoleucine, phénylalanine, 3,4-dihydroxyphénylalanine, Serine, ornithine, thréonine, glutathion.

6. Agents pour teindre des fibres contenant de la kératine contenant au moins un dérivé d'isatine de formule I et au moins une amine aromatique de formule II : 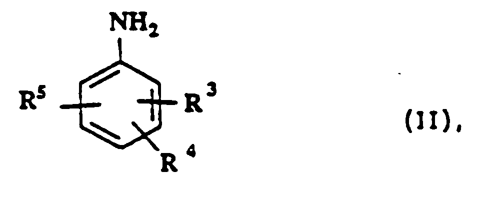 où R³, R⁴, R⁵ représentent des atomes d'hydrogène, des groupes alkyle en C₁-C₄, des groupes hydroxy, des groupes carboxyle, des groupes sulfo, des groupes aminoalkyle en C₁-C₄, ou des groupes NR⁶R⁷, ou R⁶ et R⁷ représentent indépendamment l'un de l'autre des atomes d'hydrogène, des groupes alkyle en C₁-C₄, des groupes aryle ou des groupes hydroxyalkyle en C₂-C₄, à la condition qu'au plus deux des groupes R³, R⁴, R⁵ représentent simultanément des atomes d'hydrogène et/ou des groupes alkyle en C₁-C₄ ;
tandis que deux des groupes R³, R⁴ et R⁵ peuvent former aussi ensemble un cycle benzène condensé, qui est substitué le cas échéant par un alkyle en C₁-C₄, hydroxy, carboxyle, sulfo, aminoalkyle en C₁-C₄ ou amino.

7. Agents selon la revendication 6,
caractérisés en ce qu'
on choisit l'amine aromatique de formule II dans le groupe des p-toluylènediamine, 4-aminophénol, 4-amino-2-aminométhylphénol, acide 3,4-diaminobenzoique, 2,4,5,6-tétraaminobenzène, 1-(β-Hydroxyéthyl)-2,5-diaminobenzène.

8. Agents pour teindre des fibres contenant de la kératine comprenant au moins un dérivé d'isatine de formule I et au moins une amine aromatique de formule III 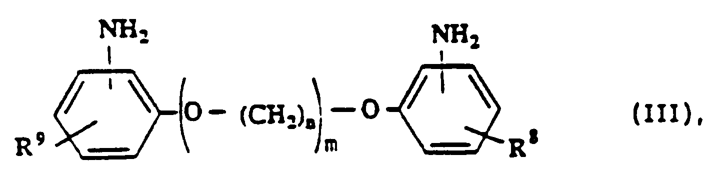 où R⁸ et R⁹, indépendamment l'un de l'autre représentent des atomes d'hydrogène, des groupes alkyle en C₁-C₄, des groupes hydroxy ou des groupes amino, n est un nombre entier de 2 à 4 et m est un nombre entier de 1 à 4.

9. Agents selon la revendication 8,
caractérisés en ce que
l'amine aromatique de formule III est le 1,3-bis-(2,4-diaminophénoxy) propane.

10. Agents pour teindre des fibres contenant de la kératine comprenant au moins un dérivé d'isatine de formule I et au moins une aminopyrimidine de formule IV : 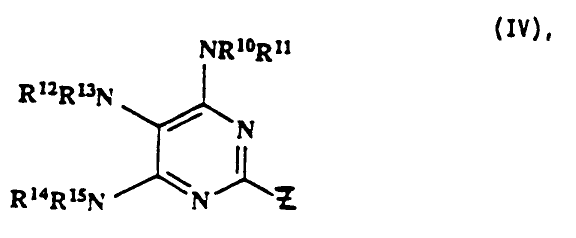 où R¹⁰, R¹¹, R¹², R¹³, R¹⁴ et R¹⁵ représentent indépendamment l'un de l'autre des atomes d'hydrogène, des groupes alkyle en C₁-C₄ ou des groupes hydroxyalkyle en C₂-C₄ et Z est l'hydrogène, un groupe OH ou NR¹⁶R¹⁷, dans lequel R¹⁶ et R¹⁷ représentent indépendamment l'un de l'autre des atomes d'hydrogène, des groupes alkyle en C₁-C₄ ou des groupes hydroxyalkyle en C₂-C₄.

11. Agents selon la revendication 10,
caractérisés en ce que
dans la tétraaminopyrimidine de formule IV les groupes R¹⁰ à R¹⁷ représentent des atomes d'hydrogène.

12. Agents pour teindre des fibres contenant de la kératine comprenant au moins un dérivé d'isatine de formule I et au moins un dérivé d'indole de formule Va : 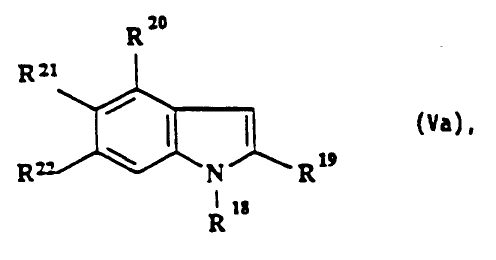 ou R¹⁸ représente un atome d'hydrogène, un groupe alkyle en C₁-C₄ ou acyle en C₂-C₄, R¹⁹ représente un atome d'hydrogène ou un groupe carboxyle et R²⁰, R²¹ et R²² représentent des atomes d'hydrogène, des groupes alkyle en C₁-C₄, des groupes acyloxy en C₂-C₄, des groupes hydroxy, des groupes amino ou des groupes alcoxy en C₁-C₄, à la condition qu'au plus deux des groupes R²⁰, R²¹, R²² représentent simultanément des atomes d'hydrogène et/ou des groupes alkyle en C₁-C₄, ou au moins un dérivé d'indole de formule Vb : 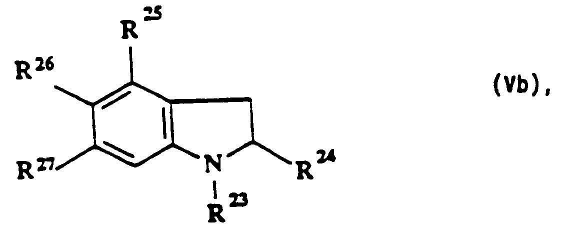 ou R²³ représente un atome d'hydrogène, un groupe alkyle en C₁-C₄ ou un groupe acyle en C₂-C₄, R²⁴ est un atome d'hydrogène ou un groupe carboxyle et R²⁵, R²⁶ et R²⁷ représentent des atomes d'hydrogène, des groupes alkyle en C₁-C₄, des groupes acyloxy en C₂-C₄, des groupes hydroxy, des groupes amino ou des groupes alcoxy en C₁-C₄, à la condition qu'au plus deux des groupes R²⁵, R²⁶, R²⁷ représentent simultanément des atomes d'hydrogène et/ou des groupes alkyle en C₁-C₄.

13. Agents selon la revendication 12,
caractérisés en ce que
le dérivé d'indole de formule Va ou le dérivé d'indoline de formule Vb est choisi dans le groupe des 5,6-dihydroxyindole, 5,6-diacétoxyindole, 4-hydroxyindoline, 6-aminoindoline et 5,6-dihydroxyindoline.

14. Agents de teinture capillaire sous forme de poudre contenant au moins un dérivé d'isatine de formule I en une quantité de 1 à 90 % en poids, de préférence 20 à 50 % en poids, et un aminoacide ou un oligopeptide constitué de 2 à 9 aminoacides en une quantité de 1 à 90 % en poids, de préférence 20 à 50 % en poids, respectivement par rapport à la totalité de l'agent de teinture en poudre.

15. Agents de teinture capillaire selon la revendication 14,
caractérisés en ce qu'
on choisit le dérivé d'isatine de formule I parmi les acide isatine-5-sulfonique, acide isatine-4-carboxylique et acide isatine-5-carboxylique et on choisit l'aminoacide ou l'oligopeptide parmi les arginine, cystéine, méthionine, proline, tyrosine, valine, glycine, acide glutamique, histidine, acide asparagique, alanine, tryptophane, cystine, lysine, hydroxyproline, leucine, Isoleucine, phénylalanine, 3,4-dihydroxyphénylalanine, seine, ornithine, thréonine, glutathion.

16. Agents de teinture capillaire sous forme d'une poudre contenant au moins un dérivé d'isatine de formule I en une quantité de 1 à 90 % en poids, de préférence 20 à 50 % en poids, et au moins une amine aromatique de formule II en une quantité de 1 à 90 % en poids, de préférence 20 à 50 % en poids, respectivement par rapport à la totalité de l'agent de teinture en poudre.

17. Agents de teinture capillaire selon la revendication 16,
caractérisés en ce qu'
on choisit le dérivé d'isatine de formule I parmi les : acide isatine-5-sulfonique, acide isatine-4-carboxylique et acide isatine-5-carboxylique et l'amine aromatique de formule II parmi les p-toluylènediamine, 4-aminophénol, 4-amino-2-aminométhylphénol, acide 3,4-diaminobenzoïque, 2,4,5,6-tétraaminobenzène, 1-(β-hydroxyéthyl)-2,5-diaminobenzène.

18. Agents de teinture sous forme d'une poudre comprenant au moins un dérivé d'isatine de formule I en une quantité de 1 à 90 % en poids, de préférence 20 à 50 % en poids et au moins une amine aromatique de formule III en une quantité de 1 à 90 % en poids, de préférence 20 à 50 % en poids, respectivement par rapport à la totalité de l'agent en poudre.

19. Agents de teinture selon la revendication 18,
caractérisés en ce qu'
on choisit le dérivé d'isatine de formule I parmi les : acide isatine-5-sulfonique, acide isatine-4-carboxylique et acide isatine-5-carboxylique et l'amine aromatique de formule III est le 1,3-bis-(2,4-diaminophénoxy)-propane.

20. Agents de teinture sous forme d'une poudre contenant au moins un dérivé d'isatine de formule I en une quantité de 2 à 90, de préférence 20 à 50 % en poids, et au moins une aminopyrimidine de formule IV en une quantité de 1 à 90 % en poids, de préférence 20 à 50 % en poids, respectivement par rapport à la totalité de l'agent de teinture en poudre.

21. Agents de teinture capillaire selon la revendication 20,
caractérisés en ce qu'
on choisit le dérivé d'isatine de formule I parmi les acide isatine-5-sulfonique, acide isatine-4-carboxylique et acide isatine-5-carboxylique et l'aminopyrimidine de formule IV est la tétraaminopyrimidine non substituée.

22. Agents de teinture capillaire sous forme d'une poudre contenant au moins un dérivé d'isatine de formule I en une quantité de 1 à 90 % en poids, de préférence 20 à 50 % en poids, et au moins un dérivé d'indole de formule Va ou un dérivé d'indoline de formule Vb en une quantité de 1 à 90 % en poids, de préférence 20 à 50 % en poids, respectivement par rapport à la totalité de l'agent de teinture en poudre.

23. Agents de teinture capillaire selon la revendication 22,
caractérisés en ce que
le dérivé d'isatine de formule I est choisi parmi les acide isatine-5-sulfonique, acide isatine-4-carboxylique et acide isatine-5-carboxylique et on choisit le dérivé d'indole de formule Va ou le dérivé d'indoline de formule Vb parmi les 5,6-dihydroxyindole, 5,6-diacétoxyindole, 4-hydroxyindoline, 6-aminoindoline et 5,6-dihydroxyindoline.
